(19) Europäisches Patentamt
European Patent Office
Office européen des brevets

(11) Numéro de publication: 0 283 333 B1

(12) **FASCICULE DE BREVET EUROPEEN**

(45) Date de publication du fascicule du brevet: **23.05.90**

(51) Int. Cl.$^5$: **C07C 69/96**, C07C 68/02

(21) Numéro de dépôt: **88400263.5**

(22) Date de dépôt: **05.02.88**

(54) **Nouveaux chloroformiates à structures vinylique, leur procédé de préparation et leurs applications.**

(30) Priorité: **13.02.87 US 14286**

(43) Date de publication de la demande: **21.09.88 Bulletin 88/38**

(45) Mention de la délivrance du brevet: **23.05.90 Bulletin 90/21**

(84) Etats contractants désignés: **CH DE FR GB IT LI**

(56) Documents cités: **EP-A- 0 040 153** **EP-A- 0 216 659**

(73) Titulaire: **SOCIETE NATIONALE DES POUDRES ET EXPLOSIFS, 12, quai Henri IV, F-75181 Paris Cédex 04(FR)**

(72) Inventeur: **Bowman, Mark, 134 East Foster Avenue Appartment 305, State College Pennsylvania 16801(US)**
Inventeur: **Olofson, Roy A., 529 Cricklewood drive, State College Pennsylvania 16802(US)**
Inventeur: **Malfroot, Thierry, 17 Chemin des Jardins, F-91100 Saintry Sur Seine(FR)**
Inventeur: **Senet, Jean-Pierre, 79 rue de la Gare Herbeauvilliers-Buthiers, F-77760 La Chapelle La Reine(FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



ACTORUM AG

## Description

L'invention concerne de nouveaux chloroformiates à structure vinylique, leur procédé de préparation et leurs applications.

Les chloroformiates sont des intermédiaires très recherchés en synthèse organique dans la mesure où ils constituent une voie d'accès facile et peu dangereuse à deux grandes familles de composés, les carbonates et les carbamates, par réaction avec un alcool ou un phénol dans le premier cas, par réaction avec une amine primaire ou secondaire dans le second cas.

Si les chloroformiates à chaîne saturée sont facilement accessibles, les chloroformiates à chaîne insaturée le sont beaucoup moins et on n'en connait en fait que très peu, notamment en ce qui concerne les chloroformiates à structure vinylique, c'est-à-dire les chloroformiates comportant une insaturation de type éthylénique entre les atomes de carbone en position 1 et 2 par rapport à la fonction chloroformiate.

En pratique on connait à l'heure actuelle les chloroformiates à structure vinylique comportant au moins un atome d'hydrogène en position bêta, tels que les chloroformiates de vinyle et de méthyl-2 vinyle qui sont décrits dans le brevet US 2 377 085, le chloroformiate d'isopropényle qui est décrit dans le brevet français 2 421 866 et quelques autres décrits dans l'article intitulé "Synthesis of Enol Chloroformates" J.O.C., 43, 752 (1978), ainsi que les chloroformiates de dihalogéno-2,2 vinyle qui sont décrits dans la demande de brevet français 85.12652 déposée le 23 août 1985.

La gamme de chloroformiates à structure vinylique effectivement disponibles pour l'homme de métier est donc limitée et cette limitation réduit considérablement l'intérêt de la voie chloroformiate pour l'obtention de carbonates et de carbamates à structure vinylique qui sont des produits par ailleurs très recherchés.

Le but de la présente invention est de proposer à l'homme de métier de nouveaux chloroformiates à structure vinylique lui permettant ainsi d'élargir la gamme des carbonates et des carbamates à structure vinylique accessible par la voie chloroformiate.

L'invention concerne donc, à titre de produits nouveaux, des chloroformiates à structure vinylique représentés par la formule :

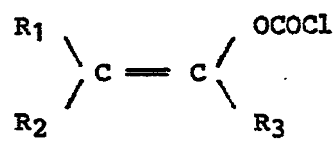

dans laquelle :
- $R_1$ et $R_2$, identiques ou différents représentent chacun un atome de chlore, un atome de brome ou un radical alkyle saturé, linéaire ou ramifié, comportant de 1 à 4 atomes de carbone, les dits radicaux $R_1$ et $R_2$ pouvant former ensemble et avec le carbone auquel ils sont liés un cycle aliphatique comportant de 4 à 8 atomes de carbone,
- $R_3$ représente :
- l'hydrogène lorsque au moins l'un des radicaux $R_1$ ou $R_2$ représente un radical alkyle,
- un radical alkyle saturé, linéaire ou ramifié, comportant de 1 à 4 atomes de carbone, le dit radical $R_3$ pouvant former avec le dit radical $R_2$ et les carbones auxquels ils sont liés un cycle hydrocarboné comportant de 5 à 8 atomes de carbone éventuellement substitué par des atomes d'oxygène formant avec les carbones du cycle la fonction cétone,
- un radical aryle éventuellement substitué par des groupes alkyle et comportant au total jusqu'à 8 atomes de carbone,
- le radical cyano - C ≡ N,
- ou un radical phosphonate de formule générale

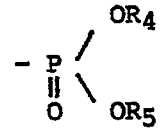

dans laquelle $R_4$ et $R_5$, identiques ou différents, représentent chacun un radical alkyle saturé, linéaire ou ramifié, comportant de 1 à 4 atomes de carbone.

L'invention concerne également un procédé de préparation des dits chloroformiates à structure vinylique caractérisé en ce que l'on fait réagir dans un milieu solvant en présence de zinc le phosgène sur un composé carbonylé alpha-halogéné de formule générale :

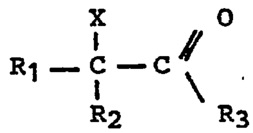

dans laquelle :
- X représente un atome de chlore ou de brome et
- $R_1$, $R_2$ et $R_3$ ont les significations précédemment indiquées.

Enfin l'invention concerne également l'application des chloroformiates à structure vinylique selon l'invention à l'obtention de carbonates et de carbamates à structure vinylique par réaction des dits chloroformiates sur un composé hydroxylé ou sur une amine primaire ou secondaire.

On décrit ci-après de manière détaillée la mise en oeuvre de l'invention.

L'invention concerne donc, à titre de produits industriels nouveaux, les chloroformiates à structure vinylique de formule :

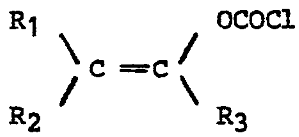

dans laquelle les radicaux $R_1$ et $R_2$, identiques ou différents, peuvent représenter chacun un atome de chlore ou un atome de brome, préférentiellement cependant un atome de chlore, ou un radical alkyle saturé, linéaire ou ramifié, comportant de 1 à 4 atomes de carbone, en particulier le radical méthyle ou le radical propyle. Les dits radicaux $R_1$ et $R_2$ peuvent former ensemble et avec le carbone auquel ils sont liés un cycle aliphatique comportant de 4 à 8 atomes de carbone, comme par exemple le cycle cyclohexyle.

Le radical $R_3$ peut avoit différentes significations :
- $R_3$ peut représenter un atome d'hydrogène lorsqu'au moins l'un des radicaux $R_1$ ou $R_2$ représente un radical alkyle.
- $R_3$ peut représenter un radical alkyle saturé, linéaire ou ramifié, comportant de 1 à 4 atomes de carbone, comme par exemple le radical méthyle. $R_3$ peut former avec $R_2$ et avec les carbones auxquels ils sont liés un cycle aliphatique comportant de 5 à 8 atomes de carbone éventuellement substitué par des atomes d'oxy gène formant avec les atomes de carbone du cycle la fonction cétone, comme par exemple le cycle cyclohexènyle ou oxo-cyclohexènyle.
- $R_3$ peut représenter un radical aryle éventuellement substitué par des groupes alkyle et comportant au total jusqu'à 8 atomes de carbone, notamment le radical phényle.
- $R_3$ peut représenter le groupe cyano - C≡N.
- $R_3$ peut enfin représenter un radical phosphonate de formule

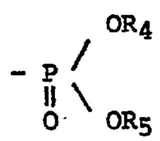

dans laquelle $R_4$ et $R_5$, identiques ou différents, représentent chacun un radical alkyle saturé, linéaire ou ramifié, comportant de 1 à 4 atomes de carbone. Les groupes phosphonates préférés sont les groupes dans lesquels $R_4$ et $R_5$ sont identiques et représentent soit le groupe méthyle, soit le groupe éthyle, c'est-à-dire les radicaux diméthylphosphonato et diéthylphosphonato.

Comme nouveaux chloroformiates à structure vinylique on peut ainsi citer le chloroformiate de méthyl-2 cyclohexényle-1, le chloroformiate de méthyl-1 dichloro-2,2 vinyle, le chloroformiate de phényl-1 dichloro-2,2 vinyle, la chlorocarbonyloxy-3 chloro-2 cyclohexène-2 one-1, le chloroformiate de méthyle-2 pentène-1 yle, le chloroformiate de cyclohexane méthylènyle, le chloroformiate de (O,O-diméthylphosphonato)-1 méthyl-2 propène-1 yle, le chloroformiate de (O,O-diéthylphosphonato)-1 méthyl-2 propène-1 yle, le chloformiate de chloro-2 propène-1 yle, le chloroformiate de méthyl-2 propène-1 yle, le chloroformiate de phényl-1 méthyl-2 propène-1 yle, le chloroformiate de cyano-1 méthyl-2 propène-1 yle.

L'invention concerne également un procédé de préparation des nouveaux chloroformiates à structure vinylique selon l'invention.

Fondamentalement le procédé selon l'invention consiste à faire réagir dans un milieu solvant en présence de zinc le phosgène sur un composé carbonylé alpha-halogéné de formule générale :

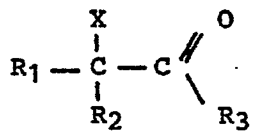

dans laquelle :

- X représente un atome de chlore ou de brome,
- $R_1$, $R_2$ et $R_3$ ont les significations précédement données.

Ces composés carbonylés sont des produits disponibles dans le commerce ou que l'on peut facilement synthétiser à partir des aldéhydes et cétones du commerce.

La réaction peut être représentée par le schéma réactionnel suivant :

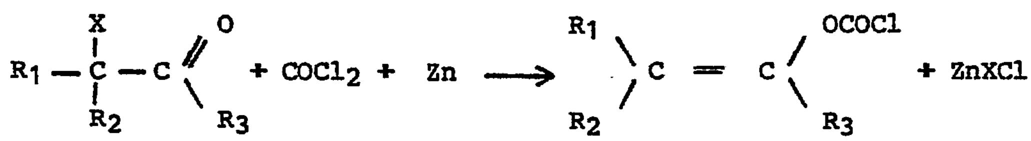

Il est surprenant d'obtenir par ce procédé des chloroformiates insaturés car il était connu que les métaux comme le zinc ou les acides de Lewis, par exemple Zn $Cl_2$, provoquaient une décomposition des chloroformiates par décarboxylation (M. Matzner et al, Chem. Review 64, pp. 668 et 670, 1964) comme par exemple :

$$2C_2H_5OCOCl + Zn \longrightarrow CH_2 = CH_2 + C_2H_5Cl + CO_2 + HCl$$

Il est également connu que les chlorures d'acide réagissent sur des cétones alpha-halogénées en présence de zinc pour former des dicétones (article de Saitkulova et al., "Zhurnal Organicheskoi Khimii" Vol 22, N° 2, pp.283-286, Feb 1986).

Il a cependant été constaté par la demanderesse que, pour que la réaction ait lieu, il est impératif que les radicaux $R_1$ et $R_2$ aient la signification donnée ci-dessus, ou autrement dit, il a été constaté que la réaction n'a pas lieu si $R_1$ ou $R_2$ représentent l'hydrogène.

Ainsi en particulier la réaction ne se produit pas si l'on utilise comme composé carbonylé le chloro-2 acétaldéhyde, le chloro-2 propanal ou la chloroacétone. Le procédé selon l'invention ne permet donc pas l'obtention des chloroformiates à structure vinylique déjà connus comme le chloroformiate de vinyle, le chloroformiate de méthyl-2 vinyle et le chloroformiate d'isopropényle.

La réaction a lieu dans un solvant, ou dans un mélange de solvants, de préférence anhydre. Les solvants sont choisis parmi les éthers linéaires, les éthers cycliques, et les esters, seuls ou en mélange avec les éthers. Le tétrahydrofuranne, le dioxanne, l'éther diéthylique, le diméthoxyéthane, l'acétate d'éthyle et l'acétate de méthyle conviennent particulièrement bien.

Selon une caractéristique essentielle de l'invention, la réaction s'effectue en présence de zinc, de préférence en poudre. Selon une variante préférée de l'invention le zinc en poudre est activé au préalable par exemple selon les méthodes indiquées par Fieser & Fieser dans "Reagents for organic synthesis", New-York, (1967), vol 1, p. 1276. On peut également recourir au zinc cuivré en poudre préparé selon R. Wilkinson dans J. Chem.Soc. (1931), p. 3057.

On emploie au moins une quantité stoechiométrique de zinc par rapport à la quantité molaire de composé carbonylé à transformer et de préférence un excès molaire compris entre 5 et 50 %.

La quantité molaire de phosgène utilisée est au moins égale à la quantité molaire de composé carbonylé à transformer, mais on préfère en général travailler en présence d'un excès de phosgène, la quantité molaire totale de phosgène utilisée étant alors comprise entre 1 et 2 fois la quantité molaire de composé carbonylé à transformer.

La température de réaction est généralement comprise entre 0°C et + 60°C, de préférence comprise entre + 5°C et + 30°C.

La durée de réaction est généralement comprise entre 30 minutes et quelques heures.

Une fois la réaction terminée, les solvants sont éliminés, par exemple par chauffage ou par aspiration sous vide. Si nécessaire les sels de zinc peuvent être précipités par exemple par addition d'un mélange 2:1 de pentane et de dioxanne. Le chloroformiate à structure vinylique peut alors être récupéré par distillation, en générale on effectuera une distillation fractionnée sous vide.

Le procédé selon l'invention permet ainsi, à partir de matières premières d'accès facile, d'obtenir de façon simple et avec de bons rendements de nouveaux chloroformiates à structure vinylique.

Les nouveaux chloroformiates à structure vinylique selon l'invention peuvent servir comme monomères pour former de nouveaux polymères par polymérisation de la fonction vinylique ou comme intermédiaires de synthèse.

L'invention concerne également certaines applications de ces nouveaux chloroformiates.

Selon ces applications, les chloroformiates à structure vinylique sont mis à réagir sur un composé hydroxylé ou sur une amine primaire ou secondaire pour former des carbonates ou des carbamates vinyliques selon les schémas réactionnels :

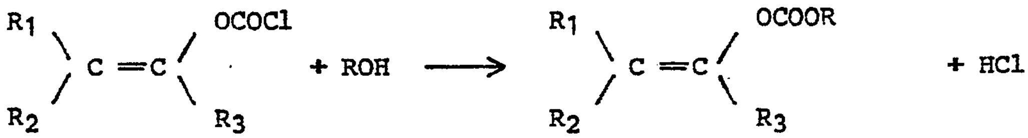

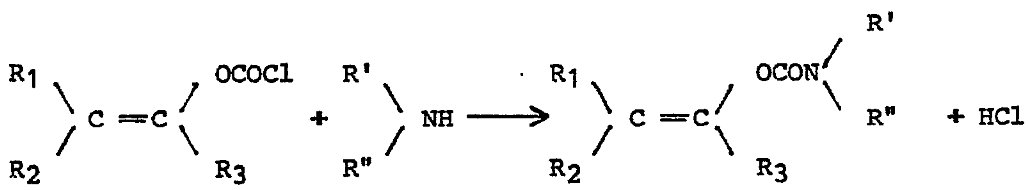

Les conditions de ces réactions sont les conditions classiques de réaction d'un chloroformiate sur un composé hydroxylé ou sur une amine (cf. Chemical Review 64, p. 651-657).

Les carbonates et les carbamates vinyliques sont des produits connus de l'homme de métier pour leurs nombreuses applications en chimie de synthèse. La préparation et l'utilisation des carbonates vinyliques sont par exemple décrites dans le brevet US 2 377 111 ou dans la demande de brevet français 86.12745. Par ailleurs la préparation et l'utilisation des carbamates vinyliques sont par exemple décrites dans le brevet français 2 533 561.

Grâce aux nouveaux chloroformiates à structure vinylique selon l'invention, les carbonates et les carbamates à structure vinylique sont obtenus de manière beaucoup plus aisée que par les procédés plus complexes de l'art antérieur.

Les exemples qui suivent illustrent, sans en limiter la portée, certaines possibilités de mise en oeuvre de l'invention.

<u>Exemple 1 :</u>

Préparation du chloroformiate de méthyl-2 cyclohexényle-1 de formule

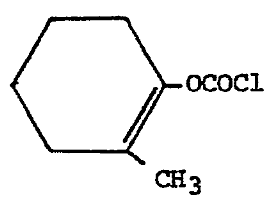

A un mélange de poudre de zinc (7,1 g soit 0,11 mole) dans 50 ml d'acétate d'éthyle refroidi dans un bain de glace on ajoute 6,5 ml (0,09 mole) de phosgène. On ajoute ensuite sous agitation 11,0 g (0,075 mole) de chloro-2 méthyl-2 cyclohexanone. Après deux heures de réaction le mélange est filtré sur filtre de silice puis lavé avec 600 ml de dichlorométhane. Le mélange ainsi obtenu est concentré puis purifié par distillation fractionnée à une température comprise entre 80°C et 82°C sous pression réduite (8 mm de mercure).

On obtient ainsi 8,9 g de chloroformiate de méthyl-2 cyclohexényle-1, présentant une pureté de 99 % par chromatographie GC ce qui correspond à un rendement de 67 %.

Le produit obtenu présente les caractéristiques suivantes :

- spectre IR (dans $CCl_4$) :
bande à 1780 $cm^{-1}$,
- spectre RMN$^1$H (dans $CDCl_3$) : δ ppm
2,4 - 1,9 (m, 7H),
1,8 - 1,4 (m, 4H),
- spectre RMN$^{13}$C (dans $CDCl_3$) : δ ppm :
148,5 (C =O),
144,4 ( =CO),
122,2 ( =C),
30,0 ; 26,3 ; 23,0, 22,1 ($CH_2$) ;
15,9 (Me).

Exemple 2 :

Préparation du chloroformiate de méthyl-1, dichloro-2,2 vinyle de formule

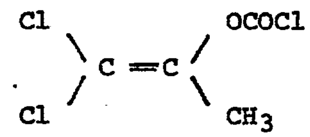

A un mélange de poudre de zinc (3,50 g soit 0,054 mole) dans 50 ml d'acétate d'éthyle refroidi dans un bain de glace on ajoute 5 ml (0,070 mole) de phosgène. On ajoute ensuite sous agitation 3,69 g (0,023 mole) de trichloro-1,1,1 acétone.

Après 4 heures de réaction le solvant a été éliminé sous vide et le produit a été extrait par lavage avec du pentane (4 x 20 ml). La solution a été concentrée et distillée à 56-58°C sous pression réduite de 17 mm de mercure.

On obtient ainsi 1,0 g de chloroformiate de méthyl-1 dichloro-2,2 vinyle présentant une pureté de 98 % par chromatographie GC, ce qui correspond à un rendement de 23 %.

Le produit obtenu présente les caractéristiques suivantes :

- spectre IR ($CCl_4$) :
1780 $cm^{-1}$,
- RMN$^1$H ($CDCl_3$) : δppm :
2,17 (s)
- RMN$^{13}$C ($CDCl_3$) : δppm :
147,3 (C =O),
143,4 ( =CO),
116,5 ($CCl_2$),
16,5 (Me).

Exemple 3 :

Préparation du chloroformiate de phényl-1 dichloro-2,2 vinyle de formule

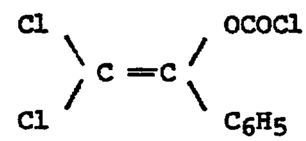

A un mélange de poudre de zinc (4,34 g soit 0,066 mole) dans 90 ml d'acétate de méthyle refroidi dans un bain de glace on ajoute 6 ml (0,084 mole) de phosgène. On ajoute ensuite en deux minutes, sous agitation, 9,80 g (0,044 mole) de trichloro-1,1,1 acétophénone. Le démarrage de la réaction est immédiat. Au bout de deux heures le solvant est éliminé par aspiration sous vide et le produit est extrait par lavage au pentane (5 x 50 ml). La solution ainsi obtenue a été concentrée et distillée à 86-88°C sous pression réduite de 0,4 mm de mercure.

On obtient ainsi 7,3 g de chloroformiate de phényl-1 dichloro-2,2 vinyle présentant une pureté de 99 % par chromatographie GC, ce qui correspond à un rendement de 66 %.

Le produit obtenu présente les caractéristiques suivantes :

- spectre IR ($CCl_4$) :
1795 $cm^{-1}$ et 1680 $cm^{-1}$,
- RMN$^1$H ($CDCl_3$) : δppm :
7,7 - 7,3 (m),
- RMN$^{13}$C ($CDCl_3$) : δppm :
147,5 (C =O),
129,8 ( =CO),
118,4 ($CCl_2$).

Exemple 4 :

Préparation du chlorocarbonyloxy-3 chloro-2 cyclohexène-2 one-1 de formule

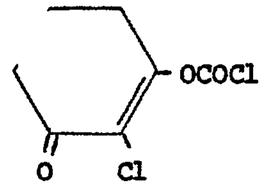

A un mélange de poudre de zinc (3,01 g soit 0,046 mole) et de phosgène (3,0 ml soit 0,042 mole) dans 40 ml d'acétate d'éthyle on ajoute, sous agitation, une solution de 6,64 g (0,037 mole) de dichloro-2,2 cyclohexanedione-1,3 dans 15 ml d'acétate d'éthyle. L'addition de la solution de dichloro-2,2 cyclohexanedione-1,3 se fait de manière fractionnée, en trois fois, pendant une durée de 1 heure. Après deux heures de réaction, le phosgène en excès est éliminé sous vide, on rajoute 40 ml d'un mélange 2:1 de pentane et de dioxane, le mélange ainsi obtenu est filtré et le filtrat est concentré sous vide. Le produit de réaction est extrait par lavage au pentane (4 x 20 ml) et distillé à 108 - 110°C sous pression réduite de 0,8 mm de mercure.

On obtient ainsi 2,3 g de chlorocarbonyloxy-3 chloro-2 cyclohexène-2 one-1, soit un rendement de 30 %.

Le produit obtenu présente les caractéristiques suivantes :
- spectre IR ($CCl_4$) :
1790 $cm^{-1}$, 1705 $cm^{-1}$, 1630 $cm^{-1}$,
- RMN$^1$H ($CDCl_3$) : δppm :
2,9 - 2,5 (m, 4H),
2,4 - 1,8 (m, 2H).

Il est à noter que la chlorocarbonyloxy-3 chloro 2 cyclohexène-2-one-1 se décompose spontanément à l'air libre à température ambiante pour donner un solide blanc identifié comme étant la dichloro-2,3 cyclohexène-2 one-1.

Exemple 5 :

Préparation du chloroformiate de méthyl-2 pentène-1 yle de formule

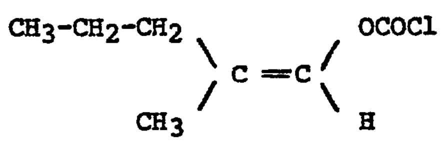

A un mélange de poudre de zinc (4,49 g soit 0,069 mole) et de phosgène (7,0 ml soit 0,098 mole) dans 70 ml d'acétate d'éthyle on ajoute sous agitation, 9,16 g (0,068 mole) de chloro-2 méthyl-2 pentanal. L'addition se fait de manière fractionnée, en trois fois, pendant deux heures. L'agitation est maintenue pendant trois heures puis on ajoute 70 ml d'un mélange 2:1 de pentane et de dioxane. Après filtration le filtrat est concentré sous vide et ensuite purifié par distillation fractionnée. On obtient ainsi 3,0 g de chloroformiate de méthyl-2 pentène-1 yle ce qui correspond à un rendement de 27 %.

Le produit obtenu présente les caractéristiques suivantes :
- point d'ébullition :
67-68°C sous 15 mm de mercure,
- spectre IR ($CCl_4$) :
3090 $cm^{-1}$, 1780 $cm^{-1}$, 1680 $cm^{-1}$,
- RMN$^1$H ($CDCl_3$) : θppm :
6,9 - 6,7 (m, 1H),
2,3 - 0,7 (m, 10H).

Exemple 6 :

Préparation du chloroformiate de cyclohexane méthylènyle de formule

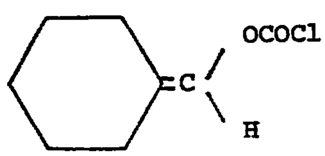

A un mélange de poudre de zinc (4,13 g) et de phosgène (5,5 ml) dans 35 ml d'acétate d'éthyle on ajoute, sous agitation, une solution de 7,0 g (0,048 mole) de chloro-1 cyclohexanecarboxaldéhyde dans 15 ml d'acétate d'éthyle. L'addition se fait de manière fractionnée, en deux fois, pendant deux heures. On rajoute alors 1,02 g de poudre de zinc et après 3 heures d'agitation le mélange est avec 30 ml d'un mélange 2:1 de pentane et de dioxanne. Après filtration, le filtrat est concentré sous vide. On rajoute alors 20 ml de pentane et on recommence les opérations de filtration et de concentration pour finir une distillation à 48-50°C sous pression de 0,7 mm de mercure.

On obtient ainsi 5,0 g de chloroformiate de cyclohexane méthylènyle ce qui correspond à un rendement de 59 %.

Le produit obtenu présente les caractéristiques suivantes :

- spectre IR ($CCl_4$) :
3090 $cm^{-1}$, 1775 $cm^{-1}$, 1680$cm^{-1}$,
- RMN$^1$H ($CDCl_3$) : δppm :
6,70 : singulet 1H,
2,5 - 1,3 (m, 10H).

<u>Exemple 7 :</u>

Préparation du chloroformiate de (O,O-diméthylphosphonato)-1 méthyle-2 propène-1 yle de formule

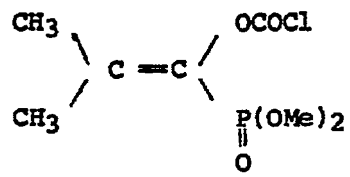

A un mélange de poudre de zinc (1,55 g soit 0,024 mole) dans 25 ml d'acétate d'éthyle on ajoute 2,5 ml (0,035 mole) de phosgène. Ensuite on rajoute 4,67 g (0,018 mole) de O,O-diméthylphosphonate de bromo-2 méthyl-2 propanoyle. Au bout de 30 minutes on élimine l'excès de phosgène et de solvant sous vide. On fait alors précipiter les sels de zinc par addition de 25 ml d'un mélange 2:1 de pentane et de dioxanne. Le mélange obtenu est alors filtré et le filtrat est concentré sous vide (0,7 mm de mercure) pendant deux heures. On obtient ainsi le chloroformiate de (O,O-diméthylphosphonato)-1 méthyl-2 propène-1 yle avec un rendement de 83 % déterminé par RMN$^1$H (solvant $CDCl_3$).

Le produit obtenu présente les caractéristiques suivantes :

- spectre IR ($CCl_4$) :
1780 $cm^{-1}$,
- RMN$^1$H ($CDCl_3$) : δppm :
3,93 (d, 6H, J = 12Hz),
2,13 (d, 3H, J = 3Hz),
1,91 (d, 3H, J = 3Hz).

<u>Exemple 8 :</u>

Préparation du chloroformiate de (O,O-diéthylphosphonato)-1 méthyl-2 propène-1 yle de formule

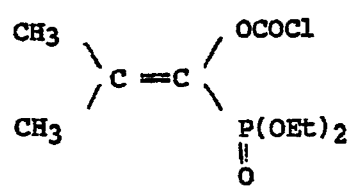

On ajoute 4,21 g (0,015 mole) de O,O-diéthylphosphonate de bromo-2 méthyl-2 propanoyle à un mélange agité de phosgène (2,0 ml soit 0,03 mole) et de poudre de zinc (1,50 g soit 0,023 mole) dans 25 ml d'acétate de méthyle. On maintient l'agitation pendant 3 heures puis on élimine le phosgène en excès sous pression réduite et on filtre. Le filtrat est concentré sous vide et on rajoute ensuite 25 ml d'un mélange 2:1 de pentane et de dioxane pour faire précipiter les sels de zinc. Le mélange est à nouveau filtré et le filtrat est concentré sous vide pendant une nuit. On obtient ainsi le chloroformiate de (O,O-diéthylphosphonato)-1 méthyl-2 propène-1 yle avec un rendement de 74% déterminé par RMN$^1$H (solvant $CDCl_3$).

Le produit obtenu présente les caractéristiques suivantes :

- spectre IR ($CCl_4$) :
1780 $cm^{-1}$ et 1640 $cm^{-1}$,
- RMN$^1$H ($CDCl_3$) : δppm :
4,34 (quintet, 4H, J = 7Hz),
2,13 (d, 3H, J = 3Hz),
1,89 (d, 3H, J = 3Hz),
1,40 (t, 6H, J = 7Hz).

Exemple 9 :

Préparation du chloroformiate de chloro-2 propène-1 yle de formule

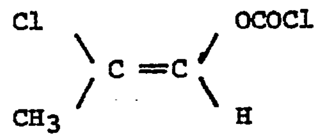

On ajoute en deux heures et demie 6 g (0,09 mole) de poudre de zinc à une solution agitée de dichloro-2,2 propanal (10,7 g soit 0,084 mole) et de phosgène (10 ml soit 0,14 mole) dans 50 ml d'acétate de méthyle. L'agitation est ensuite maintenue pendant encore 20 minutes. Le mélange est ensuite filtré et purifié par distillation fractionnée (68°C - 71°C sous 52 mm de mercure). On obtient ainsi 7,2 g de chloroformiate de chloro-2 propène-1 yle, ce qui correspond à un rendement de 56 %.

Le produit obtenu présente les caractéristiques suivantes :
- spectre IR ($CCl_4$) :
3070 $cm^{-1}$, 1780 $cm^{-1}$,
- RMN$^1$H ($CDCl_3$) : δppm :
7,2 - 7,0 (m, 1H),
2,10 (d, 1,5 H),
2,05 (d, 1,5 H).

Exemple 10 :

Préparation du chloroformiate de phényl-1 méthyl-2 propène-1 yle de formule

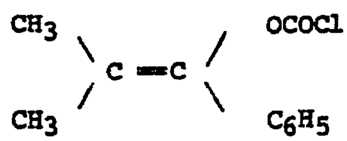

On ajoute en trois heures 3 g (0,05 mole) de poudre de zinc à une solution agitée de 4,23 g (0,023 mole) d'alpha-chloroisobutyrophénone et de 6,5 g (0,07 mole) de phosgène dans 20 ml d'acétate d'éthyle.

L'agitation est maintenue pendant une nuit et le phosgène en excès est éliminé par aspiration sous vide. Le mélange obtenu est filtré puis purifié par distillation fractionnée (54°C - 74°C sous pression réduite de 0,5 mm de mercure). On obtient ainsi le chloroformiate de phényl-1 méthyl-2 propène-1 yle qui présente les caractéristiques suivantes :
- spectre IR ($CCl_4$) :
1780 $cm^{-1}$,
- RMN$^1$H ($CDCl_3$) : δppm :
7,32 (5H),
1,83 (3H),
1,78 (3H),
- RMN$^{13}$C ($CDCl_3$) : δppm :
149,0 (C =O),
143,4 ; 133,4 ( = CH Ph),
128,9 ; 128,6 ; 128,3 ; 123,6 ($Me_2$ C =)
19,6 ; 18,2 (Me).

Exemple 11 :

Préparation du chloroformiate de méthyl-2 propène-1 yle de formule

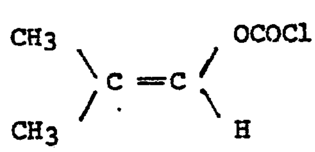

On ajoute en deux heures 15 g (0,2 mole) de poudre de zinc à une solution agitée de 14,0 g (0,13 mole) d'alphachloroisobutyraldéhyde et de 20 ml (0,26 mole) de phosgène dans 100 ml d'un mélange 2:1 d'acétate de méthyle et d'éther éthylique. On maintient l'agitation pendant une nuit et après on élimine l'excès de phosgène par aspiration sous hotte. On ajoute alors 50 ml de chloro-1 naphtalène et on récupère les produits voltails par tirage sous vide de 1 mm de mercure à une température de -78°C. Le chloroformiate de méthyl-2 propène-1 yle est isolé par distillation fractionnée (119°C - 121°C). On récupère ainsi 2,7 g de produit ce qui correspond à un rendement de 15 %.

Les caractéristiques du produit obtenu sont les suivantes :

- spectre IR ($CCl_4$) :
3085 $cm^{-1}$, 1780 $cm^{-1}$,
- RMN$^1$H ($CDCl_3$) : δppm :
7,9 - 7,6 (1H),
1,8 - 1,5 (6H).

Exemple 12 :

Préparation du chloroformiate de cyano-1 méthyl-2 propène-1 yle de formule

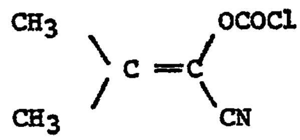

On ajoute 0,1 g de poudre de zinc à une solution de 3,5 ml (0,05 mole) de phosgène dans 25 ml d'acétate d'éthyle. Après 15 minutes d'agitation on ajoute 4,11 g (0,023 mole) de cyanure de bromo-2 méthyl-2 propanoyle et rajoute en 4 heures 2,3 g (0,03 mole) de poudre de zinc. L'agitation est maintenue pendant 30 minutes, puis le mélange est filtré, lavé avec 200 ml de dichlorométhane, concentré et distillé (80 - 83°C sous pression réduite de 10 mm de mercure).

On obtient ainsi 2,5 g de chloroformiate de cyano-1 méthyl-2 propène-1 yle ce qui correspond à un rendement de 67 %.

Les caractéristiques du produit obtenu sont les suivantes :

- spectre IR ($CCl_4$) :
2180 $cm^{-1}$, 1780 $cm^{-1}$,
- RMN$^1$H ($CDCl_3$) : δppm :
2,09 (3H),
1,90 (3H).

Exemple 13 :

Cet exemple est destiné à illustrer une application du chloroformiate de (O,O-diéthylphosphonato)-1 méthyle-2 propène-1 yle obtenu à l'exemple 8 à la synthèse d'un carbamate à structure vinylique.

L'exemple est relatif à la synthèse de la N(méthyl-2, O,O-diéthylphosphonato-1 propène-1 yl oxycarbonyl) dioctylamine par réaction du dit chloroformiate sur la dioctylamine selon le schéma réactionnel :

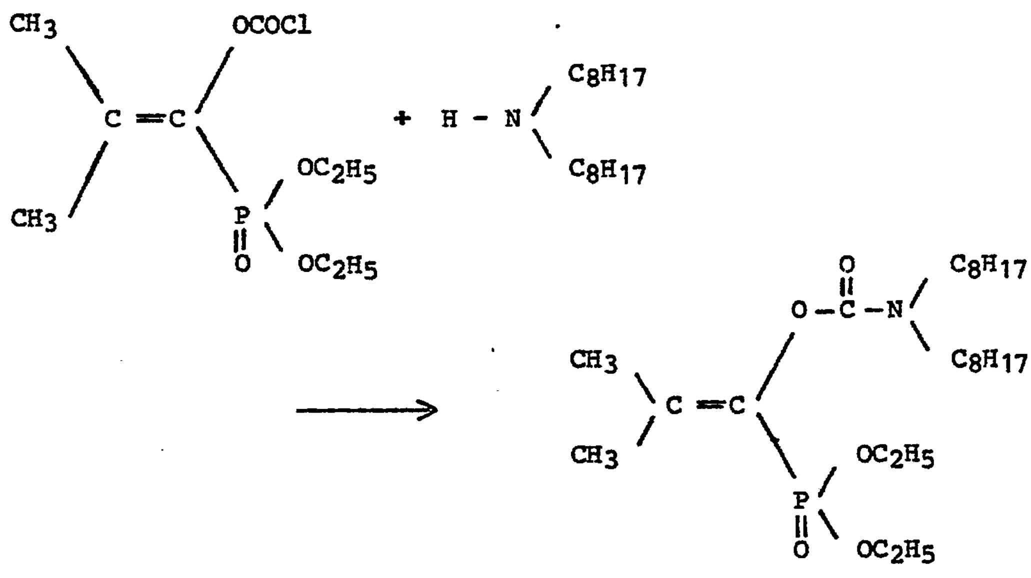

A une solution dans 10 ml de dichlorométhane de chloroformiate de (O,O-diéthylphosphonato)-1 méthyl-2 propène-1 yle obtenu comme décrit dans l'exemple 8, on rajoute sous agitation en 30 minutes une solution de 3,66 g (0,015 mole) de dioctylamine dans 5 ml de dichlorométhane.

L'agitation est ensuite maintenue pendant deux jours et le mélange obtenu est ensuite concentré, puis dilué avec 50 ml de pentane, lavé à l'eau (3 x 10 ml), lavé avec une solution aqueuse diluée d'acide chlorhydrique à 10 % (4 x 15 ml), séché avec du sulfate de potassium et reconcentré. La solution ainsi obtenue est alors filtrée et les produits volatils sont éliminés par tirage sous vide (0,7 mm de mercure) pendant une heure. On obtient ainsi 1,5 g (rendement 42 %) de N(méthyl-2, O,O-diéthylphosphonato-1 propène-1 yl oxycarbonyl) dioctyl amine.

Le carbamate obtenu a l'aspect d'une huile jaune et présente les caractéristiques suivantes :

- spectre IR ($CCl_4$) :
1710 $cm^{-1}$, 1640 $cm^{-1}$,
- RMN$^1$H ($CDCl_3$) : δppm :
4,11 (4H),
3,5 - 3,0 (4H),
2,11 (3H),
1,73 (3H),
1,8 - 0,7 (36H).

<u>Exemple 14 :</u>

Cet exemple est destiné à illustrer une application du chloroformiate de (O,O-diéthylphosphonato)-1 méthyl-2 propène-1 yle obtenu à l'exemple 8 à la synthèse d'un autre carbamate à structure vinylique.

L'exemple est relatif à la synthèse de la N(méthyl-2 O,O-diéthylphosphonato-1 propène-1 yl oxycarbonyl) morpholine selon le schéma réactionnel :

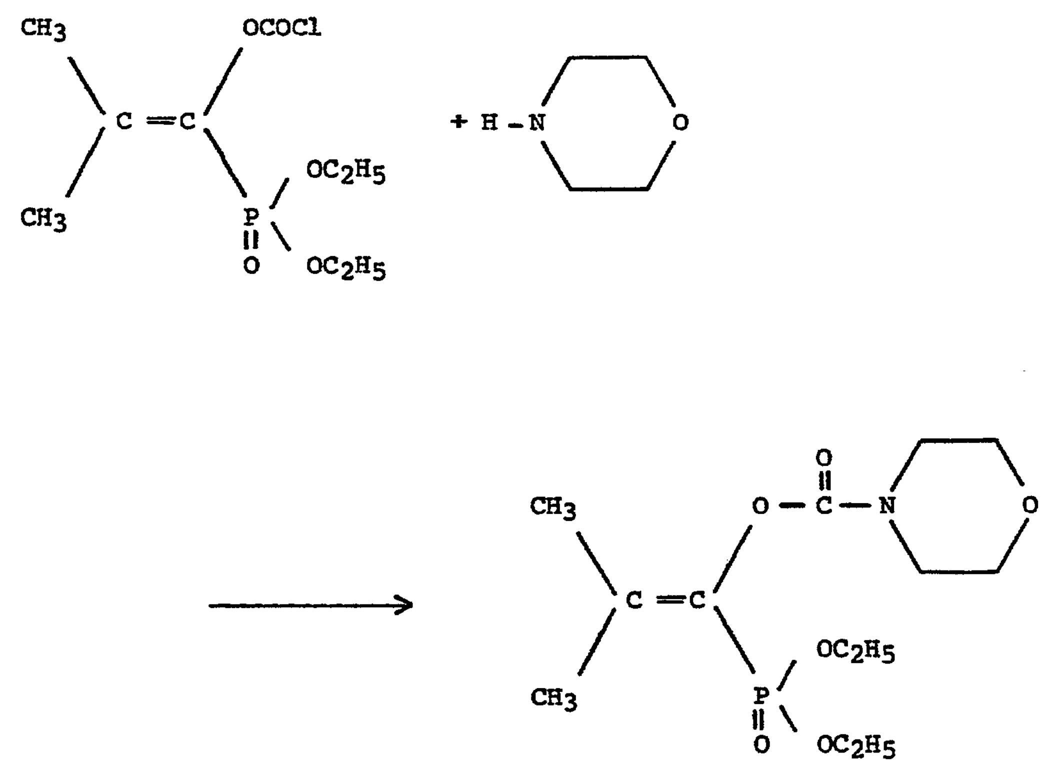

A une solution dans 30 ml d'acétate d'éthyle de chloroformiate de (O,O-diéthylphosphonato)-1 méthyl-2 propène-1 yle obtenu comme décrit dans l'exemple 8, on rajoute 4,56 g (0,052 mole) de morpholine. La réaction est immédiate et le mélange est alors filtré. Le filtrat est alors concentré et on rajoute 20 ml d'un mélange 2:1 de pentane et de dioxane pour fair précipiter les sels de zinc. Le mélange est filtré à nouveau et après concentration on obtient 4,3 g de N(méthyl-2, O,O-diéthylphosphonato-1 propène-1 yl oxycarbonyl) morpholine ce qui correspond à un rendement de 53 %.

Le carbamate ainsi obtenu à l'aspect d'une huile visqueuse et présente les caractéristiques suivantes :

- spectre IR ($CCl_4$) :
1715 $cm^{-1}$, 1645 $cm^{-1}$
- RMN$^1$H ($CDCl_3$) : δppm :
4,27 (4H),

3,8 - 3,5 (8H),
2,06 (3H),
1,80 (3H),
1,36 (3H).

**Revendications**

1. Chloroformiates à structure vinylique caractérisés en ce qu'ils sont représentés par la formule :

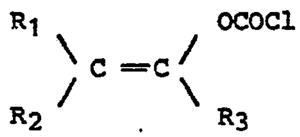

dans laquelle :
- $R_1$ et $R_2$, identiques ou différents représentent chacun un atome de chlore, un atome de brome ou un radical alkyle saturé, linéaire ou ramifié, comportant de 1 à 4 atomes de carbone, les dits radicaux $R_1$ et $R_2$ pouvant former ensemble et avec le carbone auquel ils sont liés un cycle aliphatique comportant de 4 à 8 atomes de carbone,
- $R_3$ représente :
- l'hydrogène lorsque au moins l'un des radicaux $R_1$ ou $R_2$ représente un radical alkyle,
- un radical alkyle saturé, linéaire ou ramifié, comportant de 1 à 4 atomes de carbone, le dit radical $R_3$ pouvant former avec le dit radical $R_2$ et les carbones auxquels ils sont liés un cycle hydrocarboné comportant de 5 à 8 atomes de carbone éventuellement substitué par des atomes d'oxygène formant avec les carbones du cycle la fonction cétone,
- un radical aryle éventuellement substitué par des groupes alkyle et comportant au total jusqu'à 8 atomes de carbone,
- le radical cyano - C ≡ N,
- ou un radical phosphonate de formule générale

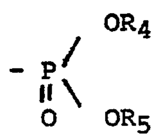

dans laquelle $R_4$ et $R_5$, identiques ou différents, représentent chacun un radical alkyle saturé, linéaire ou ramifié, comportant de 1 à 4 atomes de carbone.

2. Chloroformiates à structure vinylique selon la revendication 1 caractérisé en ce qu'ils sont représentés par la formule :

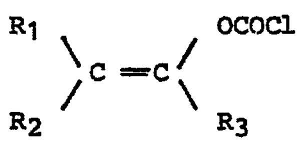

dans laquelle :
- $R_1$ et $R_2$ identiques ou différents, représentent chacun un atome de chlore ou un radical alkyle saturé, linéaire ou ramifié, comportant de 1 à 4 atomes de carbone, les dits radicaux $R_1$ et $R_2$ pouvant former ensemble et avec le carbone auquel ils sont liés le cycle cyclohexyle,
- et $R_3$,
- représente l'hydrogène lorsque au moins l'un des radicaux $R_1$ ou $R_2$ représente un radical alkyle,
- représente un radical méthyle,
- forme avec $R_2$ et les carbones auxquels ils sont liés le cycle cyclohexènyle ou oxo-cyclohexènyle,
- représente le radical phényle,
- représente le radical cyano - C ≡ N,
- ou représente un radical phosphonate de formule

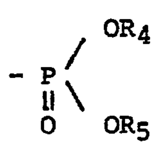

dans laquelle $R_4$ et $R_5$ sont identiques et représentent soit le groupe méthyle, soit le groupe éthyle.

3. Procédé de préparation de chloroformiates à structure vinylique selon la revendication 1 ou 2 caractérisé en ce que l'on fait réagir dans un milieu solvant en présence de zinc le phosgène sur un composé carbonylé alpha-halogéné de formule générale :

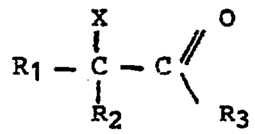

dans laquelle :
- X représente un atome de chlore ou de brome,
- $R_1$ et $R_2$ et $R_3$ ont les significations indiquées dans la revendication 1 ou 2.

4. Procédé selon la revendication 3 caractérisé en ce que le dit milieu solvant est constitué par au moins un solvant choisi dans le groupe constitué par les éthers linéaires, les éthers cycliques et les esters.

5. Procédé selon la revendication 4 caractérisé en ce que les dits solvants sont choisis dans le groupe constitué par le tétrahydrofuranne, le dioxanne, l'éther diéthylique, le diméthoxyéthane, l'acétate d'éthyle et l'acétate de méthyle.

6. Procédé selon la revendication 4 ou 5 caractérisé en ce que le milieu solvant est anhydre.

7. Procédé selon l'une quelconque des revendications 3 à 6 caractérisé en ce que le zinc est utilisé en quantité au moins stoechiométrique.

8. Procédé selon la revendication 7 caractérisé en ce que le zinc est utilisé en un excès compris entre 5 et 50 % en moles par rapport au dit composé carbonylé alpha-halogéné.

9. Procédé selon l'une quelconque des revendications 7 à 8 caractérisé en ce que le zinc utilisé est du zinc en poudre, de préférence activé, ou du zinc cuivré, en poudre .

10. Procédé selon l'une quelconque des revendications 3 à 9 caractérisé en ce les composés sont mis à réagir à une température comprise entre 0 °C et + 60 °C.

11. Procédé selon la revendication 10 caractérisé en ce que la température est comprise entre 5 °C et 30 °C.

12. Application des chloroformiates à structure vinylique selon la revendication 1 ou 2 à l'obtention de carbonates et de carbamates à structure vinylique par réaction des dits chloroformiates sur un composé hydroxylé ou sur une amine primaire ou secondaire.

## Claims

1. Chloroformates containing a vinyl structure, characterized in that they are represented by the formula:

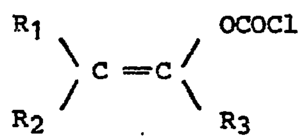

in which:
– $R_1$ and $R_2$, which may be identical or different, each represent a chlorine atom, a bromine atom or a saturated, linear or branched allkyl radical containing 1 to 4 carbon atoms, the said radicals $R_1$ and $R_2$ being able to form, together and with the carbon to which they are bonded, an aliphatic ring containing 4 to 8 carbon atoms,
– $R_3$ represents:
– hydrogen when at least one of the radicals R1 or R2 represents an alkyl radical,
– a saturated, linear or branched alkyl radical containing 1 to 4 carbon atoms, the said radical R3 being able to form, with the said radical R2 and the carbons to which they are bonded, a hydrocarbon ring containing 5 to 8 carbon atoms, optionally substituted by oxygen atoms forming the ketone function with the carbons of the ring,
– an aryl radical, optionally substituted by alkyl groups and containing in total up to 8 carbon atoms,
– the cyano radical – C = N,
– or a phosphonate radical of general formula

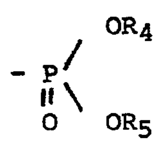

in which $R_4$ and $R_5$, which may be identical or different, each represent a saturated, linear or branched alkyl radical containing 1 to 4 carbon atoms.

2. Chloroformates containing a vinyl structure according to Claim 1, characterized in that they are represented by the formula:

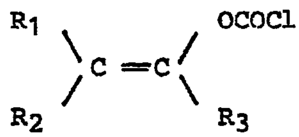

in which:

– $R_1$ and $R_2$, which may be identical or different, each represent a chlorine atom or a saturated, linear or branched alkyl radical containing 1 to 4 carbon atoms, the said radicals $R_1$ and $R_2$ being able to form, together and with the carbon to which they are bonded, the cyclohexyl ring, - and $R_3$
– represents hydrogen when at least one of the radicals $R_1$ or $R_2$ represents an alkyl radical,
– represents a methyl radical,
– form, with $R_2$ and the carbons to which they are bonded, the cyclohexenyl or oxo-cyclohexenyl ring,
– represents the phenyl radical, - represents the cyano radical - C - N, - or represents a phosphonate radical of formula

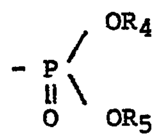

in which $R_4$ and $R_5$ are identical and represent either the methyl group or the ethyl group.

3. Process for the preparation of chloroformates containing a vinyl structure according to Claim 1 or 2, characterized in that phosgene is reacted in a solvent medium in the presence of zinc with an alpha-halogenated carbonyl compound of general formula:

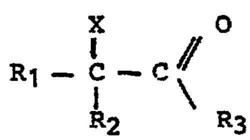

in which:

– X represents a chlorine or bromine atom,
– $R_1$ and $R_2$ and $R_3$ have the meanings indicated in Claim 1 or 2.

4. Process according to Claim 3, characterized in that the said solvent medium consists of at least one solvent chosen from the group comprising linear ethers, cyclic ethers and esters.

5. Process according to Claim 4, characterized in that the said solvents are chosen from the group comprising tetrahydrofuran, dioxane, diethyl ether, dimethoxyethane, ethyl acetate and methyl acetate.

6. Process according to Claim 4 or 5, characterized in that the solvent medium is anhydrous.

7. Process according to any one of Claims 3 to 6, characterized in that the zinc is used in at least stoichiometric amount.

8. Process according to Claim 7, characterized in that the zinc is used in an excess of between 5 and 50 mol % relative to the said alpha-halogenated carbonyl compound.

9. Process according to either of Claims 7 and 8, characterized in that the zinc used is zinc in powder form, preferably activated, or cupro-zinc in powder form. 10. Process according to any one of Claims 3 to 9, characterized in that the compounds are reacted at a temperature of between 0°C and +60°C. 11. Process according to Claim 10, characterized in that the temperature is between 5°C and 30°C.

12. Use of chloroformates containing a vinyl structure according to Claim 1 or 2 to obtain carbonates and carbamates containing a vinyl structure by reaction of the said chloroformates with a hydroxy compound or with a primary or secondary amine.

**Patentansprüche**

1. Chlorformiate mit Vinylstruktur, dadurch gekennzeichnet, daß sie die folgende Formel haben,

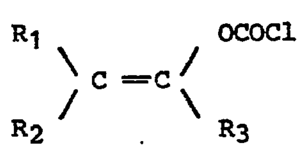

in der
– $R_1$ und $R_2$, gleich oder verschieden, je ein Chlor- oder Bromatom oder einen gesättigten, geradkettigen oder verzweigten Alkylrest mit 1–4 Kohlenstoffatomen bedeutet, wobei die Reste $R_1$, und $R_2$ zusammen mit dem Kohlenstoffatom, an das sie gebunden sind, einen aliphatischen Ring mit 4–8 Kohlenstoffatomen bilden können, und
– R3 bedeutet:
– Wasserstoff, wenn mindestens einer der Reste $R_1$ oder $R_2$ ein Alkylrest ist,
– einen gesättigten, geradkettigen oder verzweigten Alkylrest mit 1–4 Kohlenstoffatomen, der mit dem Rest $R_2$ und den Kohlenstoffatomen, an die sie gebunden sind, einen Kohlenwasserstoffring mit 5-8 Kohlenstoffatomen bilden kann, der ggfs. mit Sauerstoffatomen, die mit den Ringkohlenstoffatomen ein Keton bilden, substituiert ist,
– einen ggfs. mit Alkylresten substituierten Arylrest mit insgesamt bis zu 8 Kohlenstoffatomen,
– den Cyanorest C ≡ N,

– oder einen Phosphonatrest der allgemeinen Formel

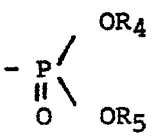

in der $R_4$ und $R_5$, gleich oder verschieden, je einen gesättigten, geradkettigen oder verzweigten Alkylrest mit 1–4 Kohlenstoffatomen bedeuten.

2. Chlorformiate mit Vinylstruktur nach Anspruch 1, dadurch gekennzeichnet, daß sie die folgende Formel haben,

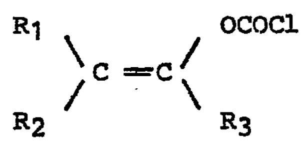

in der bedeuten:
– $R_1$ und $R_2$, gleich oder verschieden, je ein Chloratom oder einen gesättigten, geradkettigen oder verzweigten Alkylrest mit 1–4 Kohlenstoffatomen, wobei die Reste $R_1$ und $R_2$ zusammen mit dem Kohlenstoffatom, an das sie gebunden sind, einen Cyclohexylring bilden können, und
– $R_3$ Wasserstoff, wenn mindestens einer der Reste R1 oder R2 ein Alkylrest ist,
– einen Methylrest,
– mit $R_2$ und den Kohlenstoffatomen, an die sie gebunden sind, einen Cyclohexenyl- oder Oxocyclohexenylring bildet,
– einen Phenylrest,
– den Cyanorest - C ≡ N,

oder einen Phosphonatrest der Formel

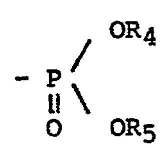

in der $R_4$ und $R_5$ gleich sind und entweder einen Methyloder Ethylrest bedeuten.

3. Verfahren zur Herstellung der Chlorformiate mit Vinylstruktur nach Anspruch 1 oder 2, dadurch gekennzeichnet, daß eine α-halogenierte Carbonylverbindung der allgemeinen Formel

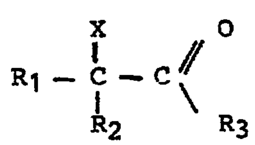

in der
– X ein Chlor- oder Bromatom ist, und
– $R_1$, $R_2$ und $R_3$ die in Anspruch 1 oder 2 angegebene Bedeutung haben,
mit Phosgen in Gegenwart von Zink in einem Lösungsmittelmedium umgesetzt wird.

4. Verfahren nach Anspruch 3. dadurch gekennzeichnet, daß als Lösungsmittelmedium mindestens ein Lösungsmittel, gewählt unter geradkettigen oder zyklischen Ethern oder Estern, verwendet wird.

5. Verfahren nach Anspruch 4, dadurch gekennzeichnet, daß als Lösungsmittel Tetrahydrofuran, Dioxan, Diethylether, Dimethoxyethan, Ethylacetat oder Methylacetat gewählt wird.

6. Verfahren nach Anspruch 4 oder 5. dadurch gekennzeichnet, daß ein wasserfreies Lösungsmittelmedium verwendet wird.

7. Verfahren nach einem der Ansprüche 3–6, dadurch gekennzeichnet, daß das Zink in einer mindestens stöchiometrischen Menge verwendet wird.

8. Verfahren nach Anspruch 7, dadurch gekennzeichnet, daß das Zink in einem Überschuß von 5 bis 50 Mol-%, bezogen auf die α-halogenierte Carbonylverbindung, verwendet wird.

9. Verfahren nach Anspruch 7 oder 8, dadurch gekennzeichnet, daß als Zink Zinkpulver, vorzugsweise aktiviert, oder kupferhaltiges Zinkpulver verwendet wird.

10. Verfahren nach einem der Ansprüche 3-9, dadurch gekennzeichnet, daß die Verbindungen bei einer Temperatur von 0 bis +60 °C umgesetzt werden.

11. Verfahren nach Anspruch 10, dadurch gekennzeichnet, daß eine Temperatur von 5 bis 30°C verwendet wird.

12. Verwendung der Chlorformiate mit Vinylstruktur nach Anspruch 1 oder 2 zur Herstellung von Carbonaten und Carbamaten mit Vinylstruktur durch Umsetzung der Chlorformiate mit einer Hydroxylverbindung oder einem primären oder sekundären Amin.